# EUROPÄISCHE PATENTSCHRIFT

(11) **EP 0 804 175 B1**
(45) Veröffentlichungstag und Bekanntmachung des Hinweises auf die Patenterteilung: **25.11.1998**
(21) Anmeldenummer: 95906301.7
(22) Anmeldetag: 05.01.1995
(51) Int. Cl.: A61K 9/70, B05C 5/02

(54) **VERFAHREN UND VORRICHTUNG ZUR HERSTELLUNG EINER FLÄCHIGEN DARREICHUNGSFORM MIT EINER ARZNEIMITTELWIRKSTOFFE ENTHALTENDEN ZUBEREITUNG**
PROCESS AND DEVICE FOR PRODUCING A FLAT DRY-ADMINISTRATION FORM WITH A PREPARATION CONTAINING ACTIVE MEDICAL AGENTS
PROCEDE ET DISPOSITIF DE FABRICATION D'UNE DOSE D'ADMINISTRATION PLATE COMPRENANT UNE PREPARATION CONTENANT DES PRINCIPES MEDICAMENTEUX ACTIFS

(30) Priorität: 13.01.1994 DE 4400769
(43) Veröffentlichungstag der Anmeldung: 05.11.1997
(73) Patentinhaber: LTS LOHMANN Therapie-Systeme GmbH, 56567 Neuwied (DE)
(72) Erfinder: GRADER, Ludwig, D-56626 Andernach (DE); SCHUMANN, Klaus, D-56567 Neuwied (DE); HILLE, Thomas, D-56564 Neuwied (DE)
(74) Vertreter: Flaccus, Rolf-Dieter, Dr.
(86) Internationale Anmeldenummer: EP9500033
(87) Internationale Veröffentlichungsnummer: WO9519163

(56) Entgegenhaltungen:
- EP-A- 0 303 025
- DE-A- 3 034 931
- DE-A- 3 423 328
- DE-A- 4 010 262
- GB-A- 1 503 511

## Beschreibung

Die Erfindung betrifft ein Verfahren sowie eine Vorrichtung zur Herstellung einer flächigen Darreichungsform mit einer Arzneimittelwirkstoffe enthaltenden Zubereitung.

Eine solche flächige Darreichungsform stellt z.B. ein dermales oder transdermales therapeutisches System in Pflasterform dar, wie z.B. in der DE 36 29 304 beschrieben. Dort ist ein Wirkstoffdepot mit textilem Flächenmaterial allseitig von einer Matrix umgeben, wobei die Dosierung des Wirkstoffes in das Depot besondere Schwierigkeiten bereitet. In der DE 36 30 603 und der EP 0 219 762 wird vorgeschlagen, die wirkstoffhaltige Masse mittels eines Walzenauftragswerkes zu beschichten. Ein ähnlicher Vorschlag wird in der DE 38 44 250 unterbreitet, wobei der Wirkstoff in fließfähiger Form auf einen bahnförmigen absorbierenden textilen Träger aufgedruckt wird. Als mögliche Druckverfahren werden die Rasterwalzenbeschichtung, die Extrusionsbeschichtung, der Siebdruck oder die Sprühbeschichtung genannt.

Diese Verfahren haben jedoch Nachteile. In einem Falle wird der Wirkstoff auf die gesamte laufende Bahn aufgetragen, obwohl in der Regel nur definierte Abschnitte des bahnförmigen Materials für die eigentliche Darreichungsform verwendet werden können, was zwangsläufig zu größeren Mengen an wirkstoffhaltigem Abfall führt.

Zum anderen ist dem Fachmann bekannt, daß die für therapeutische Systeme vorgeschriebenen Genauigkeiten nur sehr schwer oder gar nicht einzuhalten sind. Das trifft auch auf die in der DE 35 31 795 vorgeschlagenen Verfahren wie Siebdruck, Flexodruck, Tiefdruck und Inkjetting zu.

Ein praktikables Verfahren, bei dem wirkstoffhaltiger Abfall vermieden wird, ist das in der DE 37 27 214 und in der DE 37 27 232 beschriebene Tampondruckverfahren. Nachteilig ist hier, daß sowohl die in dem Druckklischee vorgelegte als auch die von dem Tampon übertragene Flüssigkeitsmenge von schwer zu beherrschenden Faktoren wie Temperatur, Viskosität, Druckgeschwindigkeit, Auflagedruck und Oberflächenbeschaffenheit abhängig ist, so daß ebenfalls die Genauigkeitsanforderungen nur schwer einzuhalten sind. Eine Kontrolle der übertragenen Menge ist nur außerhalb des eigentlichen Fertigungsprozesses durch das Bedrucken von vorher ausgewogenen Mustern möglich.

Eine Verbesserung ist durch das in P 42 30 589.6 beschriebene Verfahren möglich, wobei das bahnförmige Material durch den Tampon direkt in die befüllte Vertiefung einer Klischeeplatte gedrückt wird und dort den flüssigen Wirkstoff aufnimmt. Nachteilig bleibt auch hier die nicht exakt zu beherrschende Befüllung der Klischeeplatte sowie die Bindung an mittlere bis hohe Viskositäten der flüssigen Wirkstoffzubereitung.

Ein ganz anderer Weg wird in der DE 34 23 328 beschrieben, bei der Klebstoff und Wirkstoff räumlich voneinander getrennt mit Siebdruckwerken punktförmig aufgedruckt werden. Dieses Verfahren hat aber die Nachteile, daß einmal die bedruckte Fläche nur einen Bruchteil der Gesamtfläche ausmacht und daß zum anderen bezüglich der Genauigkeit die o.g. Einschränkungen gelten.

Ziel der vorliegenden Anmeldung ist es daher, eine fließfähige wirkstoffhaltige Zubereitung auf eine einfache und zuverlässige Art über einen breiten Viskositätsbereich mit hoher Genauigkeit und hoher Geschwindigkeit flächenmäßig definiert auf eine Schicht einer flächigen Darreichungsform zu übertragen.

Diese Aufgabe wird erfindungsgemäß mit einem Verfahren gemäß dem Kennzeichen des Patentanspruchs 1 gelöst. Zweckmäßige weitere Ausgestaltungen der Erfindung sind in den Unteransprüchen beschrieben.

Durch die Verwendung einer an sich bekannten präzisen Dosierpumpe, beispielsweise einer Kolbendosierpumpe, kann eine sehr hohe Genauigkeit bei einer ebenfalls sehr hohen Taktzahl erreicht werden. Die geförderte Menge kann dabei über einen breiten Viskositätsbereich der fließfähigen Zubereitung festgelegt werden. Durch das Passieren einer Verteilerkammer und einer daran angrenzenden Verteilerplatte, die mit mindestens einem Durchlaß versehen ist, wird die fließfähige wirkstoffhaltige Zubereitung gleichmäßig auf die definierte Fläche der Darreichungsform verteilt. Durch den kurzzeitigen Kontakt der Verteilerplatte mit der flächigen Darreichungsform wird die fließfähige wirkstoffhaltige Zubereitung direkt übertragen.

Durch dieses Verfahren wird es möglich, sowohl niedrigviskose Wirkstoffzubereitungen mit einer Viskosität ab 0,1 mPas als auch hochviskose Wirkstoffzubereitungen mit einer Viskosität bis zu 10.000 mPas problemlos zu übertragen. Vorzugsweise kann das Verfahren für Viskositäten von 100 bis 10.000 mPas eingesetzt werden.

Vorteilhaft wird die fließfähige Zubereitung, die im Sinne der Erfindung auch Einkomponenten-Systeme umfaßt, auf eine saugfähige Fläche der Darreichungsform übertragen. Dabei kann es von Vorteil sein, diese durch die Verteilerplatte zunächst zu komprimieren, dann die fließfähige Wirkstoffzubereitung zu dosieren und danach die Schicht wieder zu entspannen. Die mit der wirkstoffhaltigen Zubereitung zu versehende Fläche der Darreichungsform kann auch andere charakteristische Eigenschaften haben, wie z.B. haftklebende.

Bei großen Stückzahlen der flächigen Darreichungsform ist es vorteilhaft, das Verfahren rotativ zu gestalten. Dadurch sind Fertigungsgeschwindigkeiten von 20m/min und mehr zu erreichen. Dazu werden mehrere Verteilerkammern mit den zugehörigen Verteilerplatten über den Umfang eines Zylinders angeordnet. Mit einer oder mehreren Dosierpumpen wird die fließfähige Zubereitung nacheinander in die einzelnen Verteilerkammern gefördert, auf den Verteilerplatten vordosiert und rotativ auf die flächigen Darreichungsformen übertragen.

Ein wesentlicher Vorzug des erfindungsgemäßen Verfahrens besteht darin, daß die von der Pumpe geförderten Mengen der Produktionssicherheit entsprechend überwacht werden können. So kann mit einem Massen- oder Volumendurchflußmeßgerät - z.B. nach dem Prinzip der Coriolis-Kraft - eine Messung der Menge an dosierter fließfähiger Zubereitung an jeder einzelnen Darreichungsform damit ermittelt werden und eine 100%ige Inprozeßkontrolle ermöglichen.

Mit dem erfindungsgemäßen Verfahren ist es möglich, zwei oder mehrere verschiedene fließfähige wirkstoffhaltige Zubereitungen gleichzeitig auf benachbarte Flächen der Darreichungsform aufzubringen, wobei die Verteilerkammer entsprechend unterteilt ist.

In der Regel wird eine gleichmäßige Verteilung des Wirkstoffes in der flächigen Darreichungsform angestrebt. Dazu ist es sinnvoll, die Durchlässe gleichmäßig über die Fläche der Verteilerplatte zu verteilen und ihre lichte Weite in einer Größenordnung von 0,1 bis 1 mm zu wählen. Die Gesamtfläche der Durchlässe sollte keinesfalls 10% der Fläche der Verteilerplatte übersteigen. Für besonders hohe Anforderungen an die Gleichmäßigkeit sollte die Verteilung zweistufig aufgebaut werden, d.h. daß hinter der ersten Verteilerkammer und Verteilerplatte eine zweite Verteilerkammer und eine zweite Verteilerplatte angeordnet sind. Um ein Verstopfen der Durchlässe zu vermeiden, kann in die Vorrichtung ein Filter integriert werden. Besonders sinnvoll ist es, bei einer zweistufigen Verteilung den Filter mit der ersten Verteilerplatte zu kombinieren.

Für einige Anwendungen kann es zweckmäßig sein, die Durchlässe in der Verteilerplatte in der Form von Schlitzen auszubilden. Im übrigen kann die Verteilerplatte aus einem offenporigen gesinterten oder geschäumten Material oder aus einem Sieb besteht.

Zur Einstellung eines verarbeitbaren Viskositätsbereiches der Zubereitung ist es manchmal zweckmäßig, die Auftragsvorrichtung temperierbar auszuführen.

Die mit der Erfindung erzielbaren Vorteile bestehen insbesondere darin, daß die Dosierung der fließfähigen, wirkstoffhaltigen Zubereitung über einen breiten Viskositätsbereich und mit einer sehr hohen Genauigkeit erfolgen kann. Die dosierte Masse ist für jede einzelne Darreichungsform oder aber zumindest als Mittelwert direkt meßbar. Wird das Verfahren rotativ eingesetzt, sind sehr hohe Herstellgeschwindigkeiten möglich.

Die Erfindung ist in der Zeichnung in Ausführungsbeispielen gezeigt und wird anhand dieser im folgenden erläutert. Es zeigt:

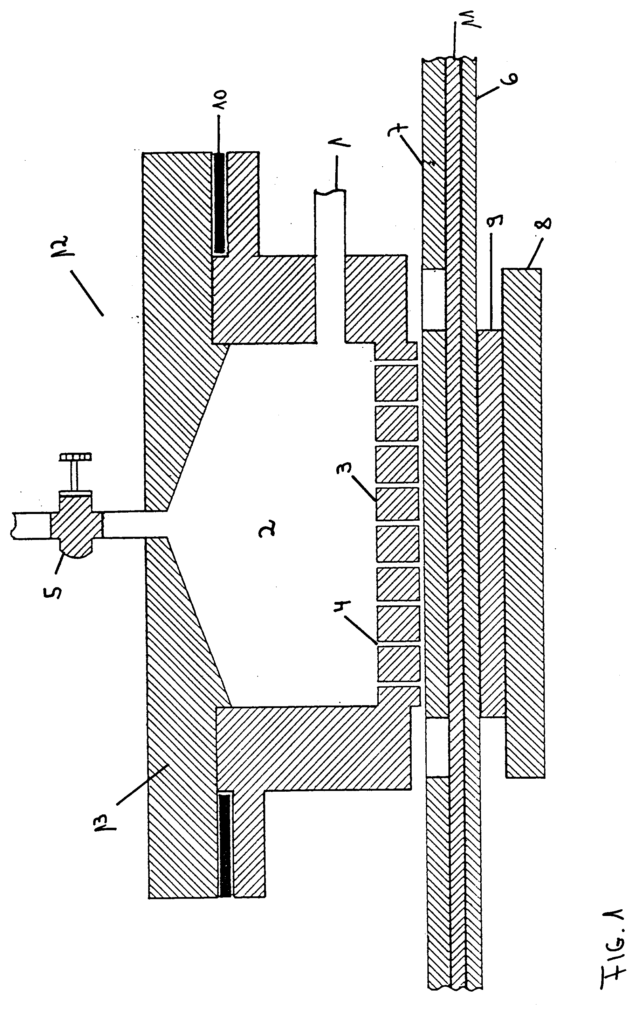

**Fig. 1** Eine Durchführungsform des erfindungsgemäßen Verfahrens in einer Schnittdarstellung

In Fig. 1 bezeichnet 1 die Zulauföffnung, durch die die zu übertragende flüssige Zubereitung von der hier nicht dargestellten Dosierpumpe in die Verteilerkammer 2 der Auftragsvorrichtung 12 gefördert wird. 3 bezeichnet die Verteilerplatte mit Durchlässen 4. Diese sind im gezeigten Beispiel als Bohrungen mit einem Durchmesser von 0,3 mm ausgeführt. 5 bezeichnet eine verschließbare Entlüftungsöffnung. 11 bezeichnet ein bahnförmiges, selbstklebendes Material auf einer wiederablösbaren Schutzschicht 6, auf dem saugfähige Vliesscheiben 7 liegen. Auf diese Schicht der späteren flächigen Darreichungsform wird die flüssige Zubereitung nach dem Durchströmen der Durchlässe 4 der Verteilerplatte 3 übertragen. Diese Übertragung geschieht durch eine Relativbewegung zwischen der Auftragsvorrichtung 12 und einer Gegendruckplatte 8,9, welche zur Abstützung des bahnförmigen Materials 6,7,11 darunter angeordnet ist. Die Gegendruckplatte besteht aus einer Stahlplatte 8 und einer darauf aufgeklebten Gummiplatte 9 mit einer Härte von vorzugsweise 85 Shore. 10 bezeichnet die Dichtung des Verschlußdeckels 13 der Verteilerkammer 2. Für die Durchführung des Verfahrens ist es ebensogut möglich, die Auftragsvorrichtung 12 feststehend anzuordnen und die Gegendruckplatte 8,9 zu bewegen. Weiterhin ist es unerheblich, ob die Übertragung der Flüssigkeit von oben oder von unten auf die Bahn 6,7,11 erfolgt.
- 1: = Zulauföffnung
- 2: = Verteilerkammer
- 3: = Verteilerplatte
- 4: = Durchlässe
- 5: = Entlüftungsöffnung
- 6: = wieder ablösbare Schutzschicht
- 7: = Vliesscheibe
- 8: = Unterlage
- 9: = Gummiplatte
- 10: = Dichtung des Dosierkopfes
- 11: = bahnförmiges, selbstklebendes Material
- 12: = Auftragsvorrichtung
- 13: = Verschlußdeckel

## Patentansprüche

1. Verfahren zur Herstellung einer flächigen Darreichungsform mit einer Arzneimittelwirkstoffe enthaltenden fließfähigen Zubereitung, dadurch gekennzeichnet, daß eine mit großer Genauigkeit dosierbare Menge der fließfähigen Zubereitung durch eine mit mindestens einem Durchlaß (4) versehene Verteilerplatte (3) einer Auftragsvorrichtung (12) auf eine mit dieser in Kontakt bringbare Schicht (7) der flächigen Darreichungsform flächenmäßig definiert übertragen wird.

2. Verfahren nach Anspruch 1, dadurch gekennzeichnet, daß die Dosierung der Zubereitung und deren Übertragung auf die Darreichungsform zeitgleich miteinander durchgeführt werden.

3. Verfahren nach Anspruch 1, dadurch gekennzeichnet, daß die Dosierung der Zubereitung und deren Übertragung auf die Darreichungsform zeitlich nacheinander durchgeführt wird.

4. Verfahren nach Anspruch 1, dadurch gekennzeichnet, daß die Dosis der fließfähigen wirkstoffhaltigen Zubereitung durch eine der Verteilerplatte (3) vorgeschaltete Verteilerkammer (2) gefördert wird.

5. Verfahren nach einem oder mehreren der Ansprüche 1 bis 4, dadurch gekennzeichnet, daß die Zubereitung auf eine Viskosität von 0,1 bis 10.000 mPas eingestellt wird.

6. Verfahren nach einem oder mehreren der Ansprüche 1 bis 5, dadurch gekennzeichnet, daß eine Zubereitung mit einer Viskosität von 100 bis 10.000 mPas verwendet wird.

7. Verfahren nach einem oder mehreren der Ansprüche 1 bis 6, dadurch gekennzeichnet, daß die Zubereitung auf eine saugfähige Schicht (7) der flächigen Darreichungsform aufgebracht wird.

8. Verfahren nach einem oder mehreren der Ansprüche 1 bis 6, dadurch gekennzeichnet, daß die Zubereitung auf eine haftklebende Schicht (6) der flächigen Darreichungsform aufgetragen wird.

9. Verfahren nach einem oder mehreren der Ansprüche 1 bis 8, dadurch gekennzeichnet, daß die Darreichungsform während der Dosierung der Zubereitung durch die Verteilerplatte komprimiert und nach Dosierung der Zubereitung entspannt wird.

10. Verfahren nach einem oder mehreren der Ansprüche 1 bis 9, dadurch gekennzeichnet, daß die Zubereitung nacheinander in über den Umfang eines Zylinders angeordnete Verteilerkammern gefördert, durch dazugehörende Verteilerplatten dosiert und rotativ übertragen wird.

11. Verfahren nach einem der Ansprüche 1 bis 10, dadurch gekennzeichnet, daß mit unterteilten Verteilerkammern gleichzeitig verschiedene fließfähige wirkstoffhaltige Zubereitungen auf benachbarte Flächen einer einzigen Darreichungsform dosiert werden.

12. Verfahren nach einem oder mehreren der Ansprüche 1 bis 11, dadurch gekennzeichnet, daß die jeweilig zu dosierende Menge einer Zubereitung unter Verwendung einer Masse- oder Volumenkontrollvorrichtung überwacht wird.

13. Vorrichtung zur Herstellung einer flächigen Darreichungsform für Arzneimittelwirkstoffe, zur Durchführung des Verfahrens entsprechend den Ansprüchen 1 bis 10, dadurch gekennzeichnet, daß auf einer Seite des zur Herstellung der Darreichungsformen bestimmten bahnförmigen Materials (6,7,11) eine mit einer Förderleitung (1) ausgestattete Auftragsvorrichtung (12), bestehend aus einer Verteilerkammer (2) mit einer mindestens einen Durchlass (4) aufweisenden Verteilerplatte (3), und auf der anderen Seite des bahnförmigen Materials (6,7,11) eine Gegendruckplatte (8,9) angeordnet sind, wobei Mittel zum gesteuerten Zusammendrücken von Auftragsvorrichtung (12) und Gegendruckplatte (8,9) vorgesehen sind.

14. Vorrichtung nach Anspruch 13, dadurch gekennzeichnet, daß die Verteilerplatte (3) Durchlässe (4) in gleichmäßiger Verteilung über die gesamte Fläche der Verteilerplatte (3) aufweist.

15. Vorrichtung nach Anspruch 13 oder 14, dadurch gekennzeichnet, daß die Durchlässe (4) eine lichte Weite von 0,1 bis 1 mm haben.

16. Vorrichtung zur Durchführung des Verfahrens nach den Ansprüchen 13 bis 15, dadurch gekennzeichnet, daß die Gesamtfläche der Durchlässe (4) maximal 10% der Fläche der Verteilerplatte (3) einnimmt.

17. Vorrichtung nach einem oder mehreren der vorangehenden Ansprüche, dadurch gekennzeichnet, daß hinter der Verteilerplatte (3) eine zweite Verteilerkammer und dahinter eine zweite Verteilerplatte angeordnet sind.

18. Vorrichtung nach einem oder mehreren der vorangehenden Ansprüche, dadurch gekennzeichnet, daß vor oder in der Verteilerkammer (3) ein Filter angeordnet ist.

19. Vorrichtung nach einem oder mehreren der vorangehenden Ansprüche, dadurch gekennzeichnet, daß die Verteilerplatte (3) aus einem offenporigen gesinterten oder geschäumten Material besteht.

20. Vorrichtung nach einem oder mehreren der vorangehenden Ansprüche, dadurch gekennzeichnet, daß die Verteilerplatte (3) aus einem Sieb besteht.

21. Vorrichtung nach einem oder mehreren der vorangehenden Ansprüche, dadurch gekennzeichnet, daß die Verteilerplatte (3) Schlitze aufweist.

22. Vorrichtung nach einem oder mehreren der vorangehenden Ansprüche, dadurch gekennzeichnet, daß die Auftragsvorrichtung (12) temperierbar ist.

## Claims

1. A process for the production of a flat administration form comprising a flowable preparation which contains pharmaceutical active substances, characterized in that a quantity of the flowable preparation, which can be metered with high accuracy, is transferred through a distribution plate (3), which is provided with at least one passage (4), of an applicator device (12) on a defined area of a layer (7) of the flat administration form, that can be brought into contact with said applicator device (12).

2. The process according to claim 1 characterized in that dosage of the preparation and its transfer onto the administration form are carried out isochronously.

3. The process according to claim 1 characterized in that dosage of the preparation and its transfer onto the administration form are carried out in chronological order.

4. The process according to claim 1 characterized in that the dose of the flowable active substance-containing preparation is conveyed through a distribution chamber (2) preceding the distribution plate (3).

5. The process according to one or several of claims 1 to 4 characterized in that the preparation is adjusted to a viscosity of 0.1 to 10,000 mPas.

6. The process according to one or several of claims 1 to 5 characterized in that a preparation having a viscosity of 100 to 10,000 mPas is used.

7. The process according to one or several of claims 1 to 6 characterized in that the preparation is applied on an absorbent layer (7) of the flat administration form.

8. The process according to one or several of claims 1 to 6 characterized in that the preparation is applied on a pressure-sensitive adhesive layer (6) of the flat administration form.

9. The process according to one or several of claims 1 to 8 characterized in that the administration form is compressed during dosage of the preparation by means of the distribution plate and is relaxed after dosage of the preparation.

10. The process according to one or several of claims 1 to 9 characterized in that the preparation is conveyed successively into distribution chambers arranged over the circumference of a cylinder, dosed through associated distribution plates, and transferred in a rotary manner.

11. The process according to any one of claims 1 to 10 characterized in that different flowable active substance-containing preparations are metered at the same time on adjacent areas of one single administration form by means of subdivided distribution chambers.

12. The process according to one or several of claims 1 to 11 characterized in that the respective amount of a preparation to be dosed is controlled by using a mass or volume control device.

13. A device for the production of a flat administration form for pharmaceutical active substances to carry out the process according to claims 1 to 10, characterized in that one side of the web-shaped material (6,7,11) intended for the production of the administration form is provided with an application unit (12) equipped with a conveying line (1) and consisting of a distribution chamber (2) having a distribution plate (3) provided with at least one passage (4), and that the other side of the web-shaped material (6,7,11) is provided with a counterpressure plate (8,9), wherein means for the controlled compression of the applicator (12) and counterpressure plate (8,9) are provided.

14. The device according to claim 13 characterized in that the distribution plate (3) has passages (4) which are uniformly distributed over the total surface of the distribution plate (3).

15. The device according to claim 13 or 14 characterized in that the passages (4) have an inside diameter of 0.1 to 1 mm.

16. The device according to claims 13 to 15 for carrying out the process, characterized in that the total area of the passages (4) takes a maximum of 10% of the area of the distribution plate (3).

17. The device according to one or several of the preceding claims characterized in that a second distribution chamber is arranged behind the distribution plate (3) and that behind said second distribution chamber a second distribution plate is arranged.

18. The device according to one or several of the preceding claims characterized in that a filter is arranged ahead of or within the distribution chamber (3).

19. The device according to one or several of the preceding claims characterized in that the distribution plate (3) consists of an open-cell sintered or foamed material.

20. The device according to one or several of the preceding claims characterized in that the distribution plate (3) consists of a screen.

21. The device according to one or several of the preceding claims characterized in that the distribution plate (3) is provided with slots.

22. The device according to one or several of the preceding claims characterized in that the application device (12) is temperature-controllable.

## Revendications

1. Procédé de fabrication d'une forme d'administration plate comprenant une préparation fluide contenant des principes médicamenteux actifs, caractérisé en ce qu'une quantité dosable de façon très précise de la préparation fluide est transférée par une plaque de distribution (3) pourvue d'au moins un passage (4) d'un dispositif d'application (12) sur une surface définie d'une couche (7) pouvant être mise en contact avec celle-ci de la forme d'administration plate.

2. Procédé selon la revendication 1, caractérisé en ce que le dosage de la préparation et le transfert de celle-ci sur la forme d'administration sont effectués simultanément.

3. Procédé selon la revendication 1, caractérisé en ce que le dosage de la préparation et le transfert de celle-ci sur la forme d'administration sont effectués successivement.

4. Procédé selon la revendication 1, caractérisé en ce que la dose de la préparation fluide contenant des principes actifs est transportée par une chambre de distribution (2) placée en amont de la plaque de distribution (3).

5. Procédé selon l'une ou plusieurs des revendications 1 à 4, caractérisé en ce que la préparation est réglée sur une viscosité comprise entre 0,1 et 10.000 mPas.

6. Procédé selon l'une ou plusieurs des revendications 1 à 5, caractérisé en ce qu'une préparation ayant une viscosité comprise entre 100 et 10.000 mPas est mise en oeuvre.

7. Procédé selon l'une ou plusieurs des revendications 1 à 6, caractérisé en ce que la préparation est appliquée sur une couche absorbante (7) de la forme d'administration plate.

8. Procédé selon l'une ou plusieurs des revendications 1 à 6, caractérisé en ce que la préparation est appliquée sur une couche auto-adhésive (6) de la forme d'administration plate.

9. Procédé selon l'une ou plusieurs des revendications 1 à 8, caractérisé en ce que la forme d'administration est comprimée par la plaque de distribution pendant le dosage de la préparation et détendue après le dosage de la préparation.

10. Procédé selon l'une ou plusieurs des revendications 1 à 9, caractérisé en ce que la préparation est successivement transportée dans des chambres de distribution disposées sur la périphérie d'un cylindre, dosée par des plaques de distribution associées et transférée par rotation.

11. Procédé selon l'une des revendications 1 à 10, caractérisé en ce qu'à l'aide de chambres de distribution divisées, différentes préparations fluides contenant des principes actifs sont dosèes simultanément sur des surfaces voisines d'une seule forme d'administration.

12. Procédé selon l'une ou plusieurs des revendications 1 à 11, caractérisé en ce que la quantité à doser en question d'une préparation est surveillée, en utilisant un dispositif de contrôle de masse ou de volume.

13. Dispositif de fabrication d'une forme d'administration plate de principes médicamenteux actifs pour la mise en oeuvre du procédé selon les revendications 1 à 10, caractérisé en ce que sur un côté du matériau (6, 7,11) en forme de bande destiné à la fabrication des formes d'administration est placé un dispositif d'application (12) équipé d'une conduite de transport (1), composé d'une chambre de distribution (2) avec une plaque de distribution (3) pourvue d'au moins un passage (4), et sur l'autre côté du matériau (6, 7,11) en forme de bande une plaque de contre-pression (8, 9), des moyens pour la compression commandée du dispositif d'application (12) et de la plaque de contre-pression (8, 9) étant prévus.

14. Dispositif selon la revendication 13, caractérisé en ce que la plaque de distribution (3) comporte des passages (4) répartis uniformément sur toute la surface de la plaque de distribution (3).

15. Dispositif selon la revendication 13 ou 14, caractérisé en ce que les passages (4) ont une largeur intérieure comprise entre 0,1 et 1 mm.

16. Dispositif pour la mise en oeuvre du procédé selon les revendications 13 à 15, caractérisé en ce que la surface totale des passages (4) occupe au maximum 10% de la surface de la plaque de distribution (3).

17. Dispositif selon l'une ou plusieurs des revendications précédentes, caractérisé en ce que derrière la plaque de distribution (3) est disposée une deuxième chambre de distribution et derrière celle-ci une deuxième plaque de distribution.

18. Dispositif selon l'une ou plusieurs des revendications précédentes, caractérisé en ce qu'un filtre est disposé devant ou dans la chambre de distribution (3).

19. Dispositif selon l'une ou plusieurs des revendications précédentes, caractérisé en ce que la plaque de distribution (3) est composée d'un matériau à pores ouverts fritté ou moussé.

20. Dispositif selon l'une ou plusieurs des revendications précédentes, caractérisé en ce que la plaque de distribution (3) est composée d'un tamis.

21. Dispositif selon l'une ou plusieurs des revendications précédentes, caractérisé en ce que la plaque de distribution (3) comporte des fentes.

22. Dispositif selon l'une ou plusieurs des revendications précédentes, caractérisé en ce que le dispositif d'application (12) est tempérable.
